# FASCICULE DE BREVET EUROPEEN

(11) **EP 2 809 161 B1**
(45) Date de publication et mention de la délivrance du brevet: **03.02.2016**
(21) Numéro de dépôt: 12806480.5
(22) Date de dépôt: 20.12.2012
(51) Int. Cl.: A01N 63/00, A01P 1/00

(54) **PROCEDE DE LUTTE BIOLOGIQUE CONTRE LES PSEUDOMONAS**
VERFAHREN ZU BIOLOGISCHEN BEKÄMPFUNG VON PSEUDOMONAS
PROCESS FOR THE BIOCONTROL OF PSEUDOMONAS

(30) Priorité: 20.12.2011 FR 1162098
(43) Date de publication de la demande: 10.12.2014
(73) Titulaire: Amoeba, 69008 Lyon (FR)
(72) Inventeur: PLASSON, Fabrice, F-69003 Lyon (FR); BODENNEC, Séléna, F-38630 Les Avenieres (FR)
(74) Mandataire: Cabinet Plasseraud
(86) Numéro de dépôt international: PCT/EP2012/076451
(87) Numéro de publication internationale: WO 2013/092897

(56) Documents cités:
- WO-A2-2008/043969
- WO-A2-2011/153460
- MICHEL ROLF ET AL: "Natürliche intrazelluläre Infektionen bei Acanthamoeben mit Pseudomonas aeruginosa nach ihrer Isolierung aus einer mikrobiologisch beanstandeten Trinkwasser-Hausinstallation eines Krankenhauses = Acanthamoebae isolated from a highly contaminated drinking water system of a hospital exhibited natural", ZENTRALBLATT FUER HYGIENE UND UMWELTMEDIZIN, STUTTGART, DE, vol. 196, no. 6, 1 janvier 1995 (1995-01-01), pages 532-544, XP009158898, ISSN: 0934-8859

## Description

La présente invention concerne un nouveau procédé de lutte biologique contre la présence de *Pseudomonas,* et sa prolifération.

*Pseudomonas* est une bactérie Gram négatif appartenant à la famille des *Pseudomonadaceae.* Cette bactérie est responsable chez l'homme de diverses infections cutanées, viscérales et pulmonaires, notamment les fibroses cystiques (4, 19). Ces bactéries sont capables de résister à de nombreux antiseptiques et antibiotiques (2) (7) expliquant sans doute en partie leur présence de plus en plus fréquente en milieu hospitalier où elles peuvent être isolées de l'environnement humide (éviers, siphons, vases, linge et objets de toilette, récipients contenant de l'eau etc). Certaines espèces présentent aussi un pouvoir pathogène envers les végétaux (8), les nématodes (10) et les amibes (1, 11, 16). Aussi, la surveillance et le contrôle de cette bactérie constituent une préoccupation de plus en plus importante.

De façon générale, il est connu que dans l'environnement, *Pseudomonas* présente une répartition ubiquitaire (5), puisque cette bactérie a été isolée du sol, des eaux d'égouts ou des eaux de rejets industriels, et des biofilms, caractéristiques qu'elle partage avec les amibes libres. Plusieurs bactéries potentiellement pathogènes (*Legionella pneumophila, Mycobacterium spp.,* et *Escherichia coli* 0157 :H7) ont développé des mécanismes pour survivre et se répliquer à l'intérieur des amibes libres (15). De plus, il a été démontré que les bactéries, dont *Pseudomonas,* peuvent développer différentes stratégies leur permettant d'échapper à la prédation par les amibes libres (12, 13, 18). En particulier, la formation du biofilm par *Pseudomonas aeruginosa* est un des mécanismes permettant à la bactérie d'échapper efficacement à la prédation par les amibes libres comme *Acanthamoeba polyphaga* (18). Bien qu'il soit connu que certaines amibes libres comme *Acanthamoeba* soient capables de développer une réponse chémotactique envers *Pseudomonas* et de se nourrir sur ces bactéries (17, 18), il a aussi été démontré que *Pseudomonas aeruginosa* inhibe rapidement la croissance de ces amibes et induit leur enkystement et leur mort en sécrétant des toxines (11-13, 17, 18). L'effet toxique des *Pseudomonas* a aussi été démontré sur des protozoaires ciliés (9).

Il apparaît donc clairement que les protozoaires et amibes libres constituent un élément important de l'écologie de *Pseudomonas.* De plus, la capacité de *Pseudomonas* à infecter et de survivre intracellulairement dans les protozoaires est un puissant indicateur que ces protozoaires sont des facteurs favorisant la résistance des *Pseudomonas* aux traitements biocides actuellement utilisés, comme indiqué par Michel et al., (14).

Dans ce contexte, les inventeurs ont mis en évidence, de façon totalement inattendue, que le genre amibien *Willaertia magna* éradique les bactéries *Pseudomonas.* Cet effet biocide, est doublé de la capacité de prédation déjà démontré de *Willaertia magna* envers d'autres agents amibiens qui peuvent servir de vecteur à *Pseudomonas* (3).

La présente invention a donc tout d'abord pour objet un procédé de lutte contre la prolifération des *Pseudomonas,* qui utilise des protozoaires du genre *Willaertia magna.* Les procédés conformes à l'invention n'incluent pas les méthodes de traitement appliquées au corps humain ou animal. Dans le procédé selon l'invention, c'est le plus souvent un flux gazeux ou liquide qui est traité avec des protozoaires de l'espèce *Willaertia magna.*

Le procédé selon l'invention trouve, en particulier application, dans la désinfection des réseaux de distribution d'eaux sanitaires ou d'eaux industrielles, des circuits de refroidissement des installations industrielles, ou des réseaux de climatisation. Les protozoaires pourront être directement ajoutés aux eaux ou aux liquides circulant dans les canalisations ou dans les réseaux à traiter. Il est également possible de les pulvériser, par exemple sous la forme d'une solution aqueuse en aérosol, dans les réseaux, les cheminées, les installations et les surfaces industrielles à désinfecter.

De façon avantageuse, les protozoaires utilisés dans le cadre de l'invention correspondent, à la souche déposée le 26 août 2006 sous le numéro PTA 7824 à l'ATCC, ou à la souche déposée le 26 août 2006 sous le numéro PTA 7825 à l'ATCC, ces deux souches ayant été déposées aux noms du CENTRE NATIONAL DE LA RECHERCHE SCIENTIFIQUE (CNRS) - 3 rue Michel Ange - 75794 PARIS CEDEX 16 / FRANCE - et UNIVERSITE LYON 1 CLAUDE BERNARD - 43 Boulevard du 11 Novembre 1918 - 69622 VILLEURBANNE Cedex / FRANCE.

Les protozoaires appartenant au genre *Willaertia* correspondant à la souche déposée sous le numéro **PTA 7824** à l'ATCC ou à la souche déposée sous le numéro **PTA 7825** à l'ATCC font partie intégrante de l'invention. Lesdites souches déposées PTA 7824 et PTA 7825 sont également décrites dans la publication de la demande internationale PCT WO2008/043969.

De tels protozoaires pourront donc être utilisés dans des agents désinfectants, en particulier destinés à l'élimination des bactéries *Pseudomonas* et à lutter contre la prolifération et la contamination par *Pseudomonas.*

D'autre part, l'invention a pour objet un agent désinfectant contenant des protozoaires de l'espèce *Willaertia magna.* Les protozoaires correspondant à la souche déposée sous le numéro **PTA 7824** à l'ATCC ou à la souche déposée sous le numéro **PTA 7825** à l'ATCC seront préférés. De façon avantageuse, l'agent désinfectant selon l'invention se présente sous la forme d'une solution ou d'une suspension aqueuse, par exemple dans de l'eau distillée. L'agent désinfectant pourra se présenter sous une forme pulvérisable, par exemple en aérosol ou tout autre moyen d'application.

L'activité des protozoaires de l'espèce *Willaertia magna,* inhibant la prolifération de *Pseudomonas* a été mise en évidence par les inventeurs en comparant la réplication de *Pseudomonas* chez les genres *Acanthamoeba* et *Hartmannella* utilisés en tant que modèles amibiens avec celle observée chez le genre amibien *Willaertia.* L'activité des protozoaires de l'espèce *Willaertia magna,* est aussi mise en évidence en démontrant l'effet de prédation de *Willaertia magna* sur des biofilms formés par *Pseudomonas aeruginosas.*

L'invention a également pour objet l'utilisation d'un agent désinfectant ou d'un protozoaire comme décrit plus haut comme biocide sur les *Pseudomonas.*

Etant donné le rôle essentiel joué par les amibes dans la prolifération et le maintien de *Pseudomonas* dans le milieu extérieur, le procédé et l'agent désinfectant selon l'invention présentent de nombreux avantages, en termes de coût, d'efficacité et de respect de l'environnement, notamment.

Les exemples ci-après permettent d'illustrer l'invention mais n'ont aucun caractère limitatif.

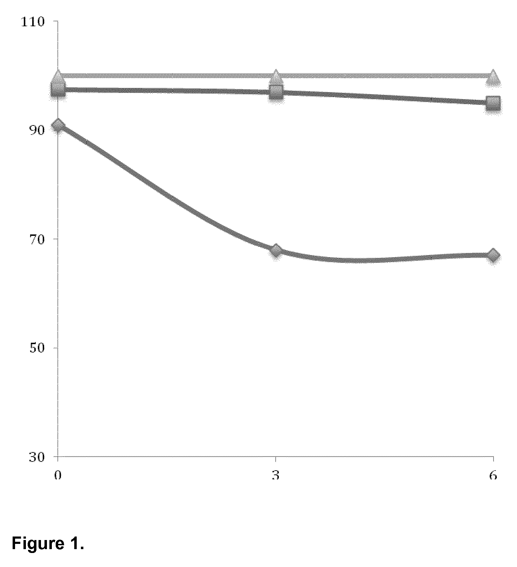

La **Figure 1** montre l'évolution spontanée des populations respectives d'amibes *Hartmannella vermiformis* (symbole « carré » ■), *Acanthamoeba castellanii* (symbole « losange » ◆) et *Willaertia* (Willaertia magna - symbole « triangle » ▲) après mise en coculture avec *Pseudomonas* à un ratio amibe/bactérie initial de 10.

Les différentes amibes libres sont mises en cocultures (temps 0 heure=T0) avec *Pseudomonas* à un ratio de 10 (10 bactéries / une amibe) comme décrit dans la partie matériels et méthodes. Des aliquotes des suspensions de coculture sont prélevés toutes les 3 heures : soit T0, T+3h, T0+6h. Le pourcentage d'amibes vivantes est déterminé par un test d'exclusion au bleu trypan et une observation microscopique à l'aide d'une cellule de Mallasez. Les données sont exprimées en % de cellules vivantes, négatives au test d'exclusion au bleu trypan.

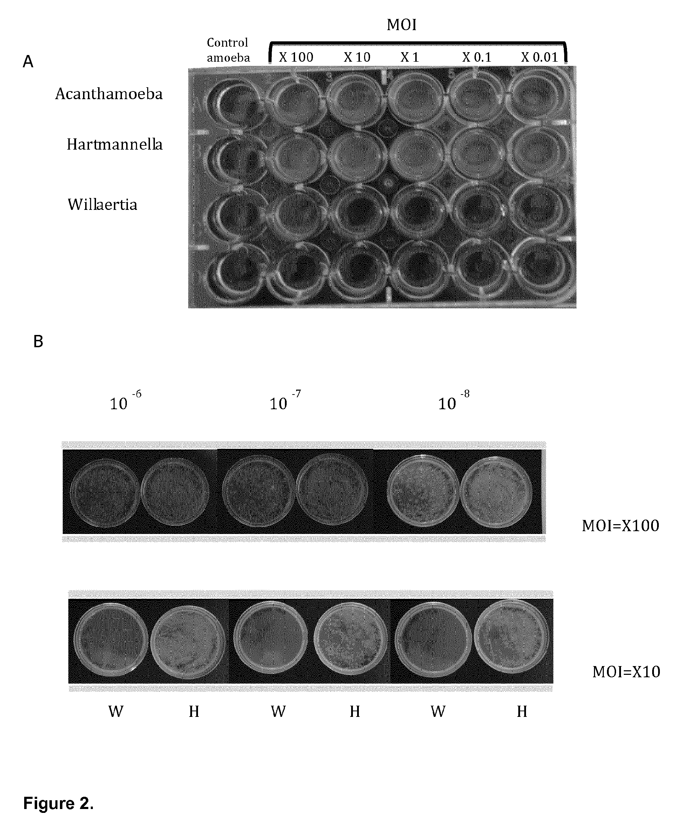

La **Figure 2** montre la croissance de *Pseudomonas* en cocultures avec *Acanthamoeba* et *Hartmannella* mais pas avec *Willaertia.*

Les *amibes* (5 x 10⁴) ont été resuspendues dans l'eau et ensemencées dans les puits. Après 1 heure, *Pseudomonas* est ajouté dans les puits de façon à atteindre différents MOI comme indiqué sur le panel A. Les cocultures sont incubées à 30°C et examinées après 24 et 48 heures. **A.** Noter l'absence de prolifération bactérienne dans le puits contenant *Willaertia* avec *Pseudomonas.* **B.** 100 µl de surnageant des puits contenants 100 bactéries/1 amibe et 10 bactéries / 1 amibe (MOI 100 et MOI 10 respectivement) sont successivement dilués (dilutions allant de 10⁻⁶ à 10⁻⁸ avec de l'eau désionisée stérile) et ensemencés sur gélose TSA. Noter l'absence de développement de colonies bactériennes dans le surnageant des cocultures avec *Willaertia magna (W),* et à l'opposé le fort développement de *Pseudomonas* en présence d*'Hartmanella* (H).

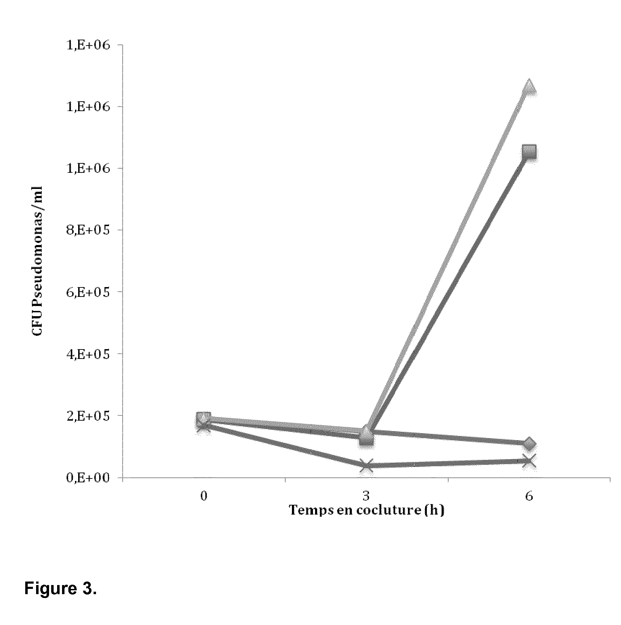

La **Figure 3** montre les cinétiques comparées du développement de *Pseudomonas* (symbole « losange » ◆) obtenues en coculture avec différents genres amibiens, dont le genre *Willaertia* (Willaertia magna - symbole « croix » ×).

Les différentes amibes libres sont séparément mises en cocultures (temps 0 heure=T0) avec *Pseudomonas* à un ratio de 10 (10 bactéries / une amibe). Des aliquotes des suspensions de coculture sont prélevés toutes les 3 heures : soit T0, T0+3h, T0+6h et les concentrations en *Pseudomonas* déterminées comme décrit dans la partie matériels et méthodes. Un témoin positif, comprenant uniquement la bactérie *Pseudomonas* en concentration équivalente à celle des cocultures servira de contrôle de la croissance de *Pseudomonas* dans le milieu. Noter la multiplication bactérienne dans les cocultures avec *Acanthamoeba castellanii* (symbole « carré » ■) et *Hartmannella vermiformis* (symbole « triangle » ▲).

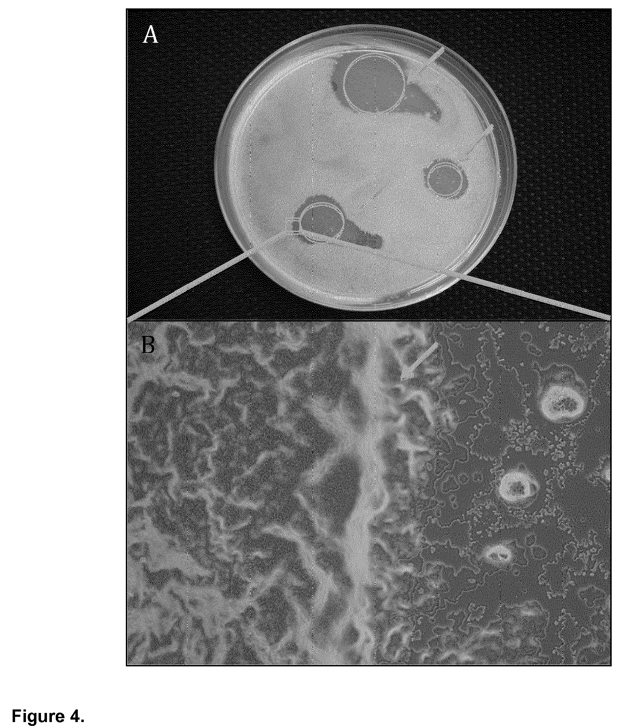

La **Figure 4** montre l'effet biocide des *Willaertia magna* sur un biofilm formé par *Pseudomonas.*

Les *Pseudomonas* et *Willaertia magna* ont été ensemencées sur gélose TSA, et incubées à 30°C pendant 24h. **A.** Les plages de lyse du biofilm de *Pseudomonas* se forment sur et au delà des dépôts de *Willaertia magna* comme indiqué par les flèches sur la Figure 4A. **B.** Front d'une plage de lyse du biofilm indiqué par la flèche. Noter à droite, le lieu de dépôt de *Willaertia magna* et l'absence de film bactérien. Noter à gauche le tapis de *Pseudomonas.*

### 1. MATERIEL ET METHODES

### 1.1. Souches utilisées:

***Pseudomonas* :** la souche utilisée est la souche CL 5210 (Oxoid, France).
- Elle est entretenue sur gélose TSA (Tryptone Soja Agar) (ref PO 5012, Oxoid, France) à raison d'un repiquage par semaine. La souche est ensemencée en stries larges sur boîte de gélose TSA et incubée 2 jours à 30°C.
- ***Amibes :*** les souches utilisées appartiennent à trois espèces amibiennes différentes :
   ∘ *Hartmannella vermiformis,*
   ∘ *Acanthamoeba castellanii, (ATCC 30010)*
   ∘ *Willaertia magna* (souches déposées à ATCC sous les n° PTA7824 et PTA 7825),

Ces trois souches sont cultivées axéniquement , en présence de 10% de sérum de veau foetal, sur milieu SCGYEM (Serum Casein Glucose Yeast Extract Medium), réparti en tubes FALCON^{®} (3033) à raison de 3ml par tube. En entretien, les formes végétatives sont repiquées tous les 8-9 jours. Pour les cocultures, on utilise des repiquages de 3 à 4 jours de manière à disposer de trophozoites en pleine phase de croissance exponentielle.

Le milieu SCGYEM est obtenu comme suit :
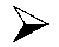 Caséine (MERCK 1.02244.010) 10 g
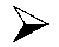 Na₂HPO₄ 1,325 g
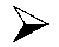 KH₂PO₄ 0,8 g
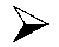 Glucose 2,5 g
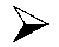 Extrait de levure (DIFCO 0127-17-9) 5 g
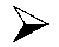 Eau distillée 900 ml
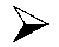 Sérum de veau foetal 100 ml

Aux 900 ml d'eau distillée, on ajoute 2,5 ml de NaOH (1 N), puis Na₂HPO₄ et KH₂PO₄. On chauffe légèrement sur plaque chauffante, puis on ajoute progressivement la caséine sous agitation magnétique. Après dissolution de la caséine, on incorpore le glucose et l'extrait de levure.

Après dissolution complète, on filtre successivement sur fibre de verre (SARTORIUS SM 6513400), puis sur membrane de 1µm (WHATMAN 7190 004). Le milieu est ensuite aliquoté en flacons de verre. Les flacons sont stérilisés à l'autoclave 20 minutes à 120°C. Avant l'utilisation définitive et répartition du milieu, on ajoute stérilement sous hotte à flux laminaire le sérum de veau foetal à raison de 10% du volume final.

### 1.2. Coculture monoamibienne de Pseudomonas.

### 1.2.1. Préparation de l'inoculum bactérien:

A partir d'une culture de 2 jours sur gélose TSA, on prépare une suspension de *Pseudomonas* dans de l'eau distillée stérile de façon à obtenir 1 unité de Densité Optique à 550nm, soit une concentration de 10⁹ UFC (Unité formant colonie) /ml.

### 1.2.2. Réalisation des cocultures mono-amibiennes

Les cocultures sont réalisées dans des tubes pour cultures cellulaires (FALCON^{®} 3033) contenant 3 ml d'eau stérilisée par autoclave. L'ensemencement des tubes se fait à raison de 1 × 10⁵ amibes/ml, à partir d'une suspension amibienne axénique préalablement dénombrée sur un hématimètre de Malassez. L'infestation des amibes par *Pseudomonas* est réalisée en fixant un ratio *Pseudomonas* / amibe de10, soit 1 × 10⁶ bactéries / ml de milieu d'incubation. Aussitôt après l'infestation, les tubes de cocultures sont centrifugés à faible vitesse (760g pendant 10 min) pour favoriser le contact entre amibes et bactéries. Après 10 min, les tubes sont remis en suspension manuellement et sont incubés, en position inclinée, à l'étuve à 30°C.

Les devenirs des amibes et de *Pseudomonas* mis en coculture sont déterminés de la façon suivante :
Les cocultures sont suivies pendant 6 heures après l'infestation bactérienne. A chaque intervalle de temps (toutes les 3 heures), les tubes de coculture sont prélevés et examinés à la fois du point de vue amibien et bactérien après agitation vigoureuse au vortex afin de détacher les amibes des parois. Pour chaque tube examiné:
   - La numération des amibes s'effectue directement sur cellule de Malassez.
   - La détermination des concentrations en *Pseudomonas* est effectuée par étalement direct du milieu de culture sur gélose TSA après dilution de 10 en 10 en eau distillée stérile, dans des microtubes Eppendorf. Chaque dilution est étalée en triplicat sur gélose TSA à raison de 100µl par boîte. Les boîtes sont alors mises à incuber à 30°C pendant 48 heures au minimum. Une première lecture des géloses TSA s'effectue 24 heures après l'étalement en dénombrant les colonies; elle est suivie d'une deuxième lecture au 2^{ème} jour pour confirmation. Les concentrations de *Pseudomonas* sont exprimées en UFC / ml de milieu d'incubation en tenant compte du facteur de dilution et en supposant que chaque colonie correspond à 1 bactérie initialement présente dans la suspension diluée.

Pour chaque genre amibien, les courbes de croissance de *Pseudomonas,* sont représentées en fonction du temps.

De plus, l'éventuel effet cytotoxique de *Pseudomonas* sur les différentes espèces d'amibes est déterminé de la façon suivante :
- en comptant la proportion d'amibes présentant une positivité au test d'exclusion au bleu trypan. Ce test est réalisé sous microscope en comptant sous cellule de Malassez le nombre de cellules positives au bleu trypan / au nombre de cellules totales.

### 1.3. Effet de Willaertia magna sur les biofilm de Pseudomonas.

Les *Willaertia* ont été déposées sur le tapis de *Pseudomonas* qui venait d'être étalé sur la gélose TSA. Les géloses sont placées à 30°C pendant 24 heures de façon à permettre le développement d'un film bactérien sur la surface de la gélose. Les géloses sont ensuite observées au microscope optique (grossissement × 400) afin d'y détecter la formation d'éventuelles plages de lyse du tapis bactérien.

### 2. RESULTATS

### 2. 1. Willaertia magna présente une résistance à Pseudomonas.

L'effet de *Pseudomonas* sur la survie des différentes espèces amibiennes testées a été déterminé par un test d'exclusion au bleu trypan. Très rapidement après mise en coculture d'*Acanthamoeba castellanii* avec la bactérie, il apparaît un effet cytotoxique majeur chez cette espèce amibienne, avec une chute de ∼ 30% de la viabilité après 3 heures de coculture (voir **Figure 1**). A l'opposé ce phénomène n'est jamais observé lorsque *Willaertia magna* est mis en coculture avec *Pseudomonas,* y compris jusqu'à 9 heures d'incubation avec une viabilité se maintenant proche des 100% **(****Figure 1****).** Comme *Willaertia magna,* l'amibe libre *Hartmanella vermiformis* ne présente pas de chute en termes de viabilité déterminée par exclusion au bleu trypan **(****Figure 1**). L'ensemble de ces observations (pas d'enkystement et pas de cytotoxicité induite par *Pseudomonas*) démontrent clairement que *Willaertia magna et Hartmanella vermiformis,* au contraire des autres espèces amibiennes du type *Acanthamoeba castellanii,* présentent la faculté originale de résister à *Pseudomonas.*

### 2.2. Prédation de Pseudomonas par Willaertia magna

Les résultats des cocultures de *Pseudomonas* réalisées en présence d'amibes appartenant aux genres *Hartmannella* et *Acanthamoeba* démontrent une importante multiplication de la bactérie en présence de ces deux genres amibiens puisqu'on note une augmentation des concentrations bactériennes en 6 heures (voir **Figure 2****).** A l'inverse, (alors que les cocultures sont réalisées dans des conditions strictement identiques), on constate en présence de l'amibe *Willaertia magna* une réduction de l'ordre d'un log des concentrations en *Pseudomonas* détectables, par rapport au témoin contenant Pseudomonas uniquement (voir **Figure 2****, et** **Figure 3****).** La chute mesurée des concentrations en *Pseudomonas,* démontre un effet massif de prédation des *Willaertia magna* envers *Pseudomonas.*

*Willaertia magna* et *Hartmannella vermiformis* survivent, mais seule *Willaertia magna* empêche la prolifération bactérienne. Cet effet des *Willaertia magna* sur les *Pseudomonas* est encore illustré **Figure 3** **et** **4****.** Après 48 heures d'incubation dans de l'eau les cocultures d'*Acanthamoeba* et *Hartmannella* avec la bactérie démontrent une prolifération de *Pseudomonas* (Noter l'aspect troublé des puits contenant *Pseudomonas* et *Hartmanella* ou *Acanthamoeba,* due à la prolifération bactérienne) **(****Figure 3****, panel A).** Les concentrations de *Pseudomonas* déterminées dans le surnageant des puits de coculture démontrent l'absence de bactéries avec *Willaertia magna* **(****Figure 3****, panel B)** lorsque celles-ci ont été incubées à un MOI de 10 (10 bactéries/ une amibe).

L'effet de prédation des *Willaertia magna* sur *Pseudomonas* est aussi démontré sur la **Figure 4****.** En effet, après 24 heures en présence des *Willaertia magna,* apparaissent très clairement des surfaces sur la gélose où le tapis bactérien a disparu (ces zones sont appelées ici plages de lyse du tapis/biofilm bactérien-**Figure 4** **panel A).** L'examen microscopique des géloses montre aussi que les *Willaertia magna* sont concentrées à la limite de cette plage de lyse; cet effet est aussi illustré **Figure 4** **panel B,** où apparaît clairement la disparition du tapis bactérien sous l'action des *Willaertia magna.* L'ensemble de ces données et observations montrent clairement l'effet de prédation de *Willaertia magna* envers la bactérie pathogène *Pseudomonas* ayant développé un biofilm.

### Références bibliographiques

1. Abd H, Wretlind B, Saeed A, Idsund E, Hultenby K, and Sandstrom G. Pseudomonas aeruginosa utilises its type III secretion system to kill the free-living amoeba Acanthamoeba castellanii. J Eukaryot Microbiol 55: 235-243, 2008.
2. Ashish A, Shaw M, Winstanley C, Ledson MJ, and Walshaw MJ. Increasing résistance of the Liverpool Epidemic Strain (LES) of Pseudomonas aeruginosa (Psa) to antibiotics in cystic fibrosis (CF)-A cause for concern? J Cyst Fibros 2011.
3. Bodennec J, Dey R, and Pernin P. Novel method for biologically combating the proliferation of Legionella pneumophila, and novel disinfecting agent containing amoebic protozoa of the Willaertia genus. edited by University CBL. France: 2010.
4. Bodey GP, Bolivar R, Fainstein V, and Jadeja L. Infections caused by Pseudomonas aeruginosa. Rev Infect Dis 5: 279-313, 1983.
5. Bredenbruch F, Geffers R, Nimtz M, Buer J, and Haussier S. The Pseudomonas aeruginosa quinolone signal (PQS) has an iron-chelating activity. Environ Microbiol 8: 1318-1329, 2006.
6. Davies B, Chattings LS, and Edwards SW. Superoxide generation during phagocytosis by Acanthamoeba castellanii: similarities to the respiratory burst of immune phagocytes. J Gen Microbiol 137: 705-710, 1991.
7. Fernandez M, Conde S, de la Torre J, Molina-Santiago C, Ramos JL, and Duque E. Mechanisms of résistance to chloramphenicol by Pseudomonas putida KT2440. Antimicrob Agents Chemother 2011.
8. Fones H, and Preston GM. Reactive oxygen and oxydative stress tolerance in plant pathogenic pseudomonas. FEMS Microbiol Lett doi: 10.1111/j.1574-6968.2011.02449.x.[Epub ahead of print]: 2011.
9. Hahn MW, Moore ER, and Hofle MG. Role of Microcolony Formation in the Protistan Grazing Defense of the Aquatic Bacterium Pseudomonas sp. MWH1. Microb Ecol39: 175-185, 2000.
10. Irazoqui JE, Troemel ER, Feinbaum RL, Luhachack LG, Cezairliyan BO, and Ausubel FM. Distinct pathogenesis and host responses during infection of C. elegans by P. aeruginosa and S. aureus. PloS Pathog 6: e1000982, 2010.
11. Julia AG, and Morgan BM. The effects of selected strains of pigmented microorganisms on small free-living amoeba. Can J Microbiol 10: 579-584, 1964.
12. Matz C, Bergfeld T, Rice SA, and Kjelleberg S. Microcolonies, quorum sensing and cytotoxicity determine the survival of Pseudomonas aeruginosa biofilms exposed to protozoan grazing. Environ Microbiol 6: 218-226, 2004.
13. Matz C, Moreno AM, Alhede M, Manefield M, Hauser AR, Givskov M, and Kjelleberg S. Pseudomonas aeruginosa uses type III secretion system to kill biofilm-associated amoebae. Isme J 2: 843-852, 2008.
14. Michel R, Burghardt H, and Bergmann H. Acanthamoeba, naturally intracellularly infected with pseudomonas aeruginosa, after their isolation from a microbiologically contaminated drinking water system in a hospital. Zentralbl Hyg Umweltmed 196: 532-544, 1995.
15. Molmeret M, Horn M, Wagner M, Santic M, and Abu Kwaik Y. Amoebae as training grounds for intracellular bacterial pathogens. Appl Environ Microbiol 71: 20-28, 2005.
16. Qureshi MN, Perez AA, 2nd, Madayag RM, and Bottone EJ. Inhibition of Acanthamoeba species by Pseudomonas aeruginosa: rationale for their selective exclusion in corneal ulcers and contact lens care systems. J Clin Microbiol 31 : 1908-1910, 1993.
17. Wang X, and Ahearn DG. Effect of bacteria on survival and growth of Acanthamoeba castellanii. Curr Microbiol 34: 212-215, 1997.
18. Weitere M, Bergfeld T, Rice SA, Matz C, and Kjelleberg S. Grazing resistance of Pseudomonas aeruginosa biofilms depends on type of protective mechanism, developmental stage and protozoan feeding mode. Environ Microbiol 7: 1593-1601, 2005.
19. Yang L, Jelsbak L, and Molin S. Microbial ecology and adaptation in cystic fibrosis airways. Environ Microbiol 13: 1682-1689, 2011.

## Revendications

1. Procédé de lutte contre la prolifération de *Pseudomonas,* à l'exception des méthodes de traitement appliquées au corps humain ou animal, **caractérisé en ce qu'**il utilise des protozoaires de l'espèce Willaertia magna.

2. Procédé selon la revendication 1 **caractérisé en ce que** l'on traite un flux gazeux ou liquide, ou une surface solide avec des protozoaires de l'espèce *Willaertia magna.*

3. Procédé selon la revendication 1 ou 2 **caractérisé en ce que** ces protozoaires correspondant, à la souche déposée sous le numéro **PTA 7824** à l'ATCC ou à la souche déposée sous le numéro **PTA 7825** à l'ATCC.

4. Procédé selon l'une des revendications 1 à 3 **caractérisé en ce qu'**il est mis en oeuvre pour la désinfection des réseaux de distribution des eaux sanitaires ou des eaux industrielles, des circuits de refroidissement des installations industrielles, ou des réseaux de climatisation.

5. Procédé selon l'une des revendications 1 à 3 **caractérisé en ce qu'**il est mis en oeuvre pour lutter contre la formation de biofilms dans les canalisations d'eau, les surfaces en contact ou non avec des denrées alimentaires humaines ou animales.

6. Utilisation d'un agent désinfectant contenant des protozoaires de l'espèce *Willaertia magna,* comme biocide sur les *Pseudomonas,* à l'exception d'une utilisation pour le traitement appliqué au corps humain ou animal.

7. Utilisation selon la revendication 6, **caractérisée en ce que** les protozoaires correspondent à la souche déposée sous le numéro **PTA 7824** à l'ATCC ou à la souche déposée sous le numéro **PTA 7825** à l'ATCC.

8. Utilisation la revendication 6 ou 7, **caractérisée en ce que** l'agent désinfectant se présente sous la forme d'une solution ou d'une suspension aqueuse.

## Patentansprüche

1. Verfahren zur Bekämpfung des Ausbreitens von Pseudomonas, mit Ausnahme der auf den menschlichen oder tierischen Körper angewandten Behandlungsmethoden, **dadurch gekennzeichnet, dass** es Protozoen der Gattung Willaertia magna verwendet.

2. Verfahren nach Anspruch 1, **dadurch gekennzeichnet, dass** man eine gasförmige oder flüssige Strömung, oder eine feste Oberfläche mit Protozoen der Gattung Willaertia magna behandelt.

3. Verfahren nach Anspruch 1 oder 2, **dadurch gekennzeichnet, dass** diese Protozoen dem Stamm entsprechen, der unter der Nummer PTA 7824 bei der ATCC hinterlegt worden ist, oder dem Stamm, der unter der Nummer PTA 7825 bei der ATCC hinterlegt worden ist.

4. Verfahren nach einem der Ansprüche 1 bis 3, **dadurch gekennzeichnet, dass** es zur Desinfektion der Versorgungsnetze für Brauchwasser oder für Industriewässer, für Kühlkreise für industrielle Anlagen oder für Klimaanlagen angewandt wird.

5. Verfahren nach einem der Ansprüche 1 bis 3, **dadurch gekennzeichnet, dass** es zur Bekämpfung der Bildung von Biofilmen in Wasserleitungen, Oberflächen, die mit menschlichen oder tierischen Nahrungsmitteln in Berührung oder nicht in Berührung kommen angewandt wird.

6. Verwendung eines Desinfektionsmittels, Protozoen der Gattung Willaertia magna umfassend, als Biozid auf den Pseudomonas, mit Ausnahme einer Verwendung zur Behandlung, die auf den menschlichen oder tierischen Körper angewandt wird.

7. Verwendung nach Anspruch 6, **dadurch gekennzeichnet, dass** die Protozoen dem Stamm entsprechen, der unter der Nummer PTA 7824 bei der ATCC hinterlegt worden ist, oder dem Stamm, der unter der Nummer PTA 7825 bei der ATCC hinterlegt worden ist.

8. Verwendung nach Anspruch 6 oder 7, **dadurch gekennzeichnet, dass** das Desinfektionsmittel in Form einer wässrigen Lösung oder Suspension vorliegt.

## Claims

1. Method for combatting the proliferation of *Pseudomonas,* with the exception of treatment methods applied to the human or animal body, **characterised in that** it uses protozoa of the *Willaertia magna* species.

2. Method according to claim 1, **characterised in that** a gaseous or liquid flow or a solid surface is treated with protozoa of the *Willaertia magma* species.

3. Method according to claim 1or 2, **characterised in that** these protozoa correspond to the strain filed under the number PTA 7824 at the ATCC or to the strain filed under the number PTA 7825 at the ATCC.

4. Method according to one of claims 1 to 3, **characterised in that** it is implemented for disinfecting systems for distributing domestic water or industrial water, cooling circuits of industrial plant, or air-conditioning systems.

5. Method according to one of claims 1 to 3, **characterised in that** it is implemented for combatting the formation of biofilms in water pipes and surfaces in contact or not with human or animal foodstuffs.

6. Use of a disinfectant containing protozoa of the *Willaertia magma* species, as a biocide on *Pseudomonas,* with the exception of use for treatment applied to the human or animal body.

7. Use according to claim 6, **characterised in that** the protozoa correspond to the strain filed under the number PTA 7824 at the ATCC or to the strain filed under the number PTA 7825 at the ATCC.

8. Use according to claim 6 or 7, **characterised in that** the disinfectant is in the form of a solution or an aqueous suspension.
